# EUROPEAN PATENT APPLICATION

(11) **EP 2 292 237 A1**
(43) Date of publication of application: **09.03.2011**
(21) Application number: 09382145.2
(22) Date of filing: 14.08.2009
(51) Int. Cl.: A61K 31/5377, A61K 45/06, A61P 25/02

(54) **Sigma ligands for the prevention or treatment of pain induced by chemotherapy**

(71) Applicant: Laboratorios del. Dr. Esteve, S.A., 08041 Barcelona (ES)
(72) Inventor: Baeyens- Cabrera, José Manuel, 18014 Granada (ES); Buschmann, Helmut Heinrich, 52076 Aachen (Walheim) (DE); Vela Hernández, José Miguel, 08028 Barcelona (ES); Zamanillo-Castanedo, Daniel, 08041 Barcelona (ES); Nieto-López, Francisco-Rafael, 18014 Granada (ES)
(74) Representative: ABG Patentes, S.L.

(57) **Abstract**

The invention refers to the use of a sigma ligand of formula (I) to prevent or treat pain induced by a chemotherapeutic agent, especially pain induced by taxanes, vinca alkaloids or platinum derivatives chemotherapeutic drugs.

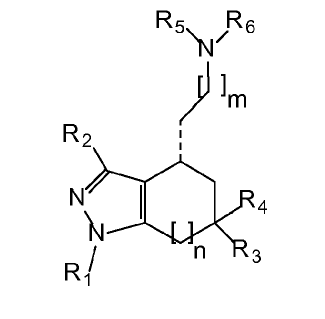

## Description

### FIELD OF THE INVENTION

The present invention relates to the use of a group of sigma receptor ligands in the prevention or treatment of pain resulting from chemotherapy. The present invention also refers to a combination of a sigma receptor ligand and a chemotherapeutic agent, and its use in the prevention or treatment of pain developing as a consequence of chemotherapy.

### BACKGROUND

The treatment of pain conditions is of great importance in medicine. There is currently a world-wide need for additional pain therapy. The pressing requirement for a specific treatment of pain conditions is documented in the large number of scientific works that have appeared recently in the field of applied analgesics.

PAIN is defined by the International Association for the Study of Pain (IASP) as "an unpleasant sensory and emotional experience associated with actual or potential tissue damage, or described in terms of such damage" (IASP, Classification of chronic pain, 2nd Edition, IASP Press (2002), 210). Although pain is always subjective, its causes or syndromes can be classified. Some of the most relevant pain subtypes are neuropathic pain, allodynia, hyperalgesia, and peripheral neuropathy.

On the other hand, cancer and its associated therapies are some of the biggest health concerns in the world. Chemotherapy, in combination with or as an alternative to surgery, is the method of choice in most cases for controlling or helping patients struck by carcinomas.

Chemotherapy is defined as the use of chemical substances to treat disease and, in the sense of this invention, refers primarily to the use of cytotoxic or cytostatic drugs, called chemotherapeutic drugs, to treat cancer. In general it is a systemic treatment. Chemotherapy in cancer treatment consists of a personalized combination of potent chemotherapy drugs, designed to slow rapid cancer tumor growth, shrink tumors, kill cancer cells, and prevent the spread of cancer. The chemotherapeutic drugs prevent cells from replicating in the typical, out-of-control manner in which cancer cells divide.

Peripheral neurotoxicity is a clinically significant complication of cancer chemotherapy. For several of the most effective drugs (e.g. taxanes, vinca alkaloids, cisplatin, bortezomib, thalidomide and lenolidamide), neurotoxicity is dose-limiting and sometimes forces the termination of otherwise successful therapy (Polomano and Bennett, Pain Med., 2001, 2(1), 8-14; Park et al., Curr. Med. Chem, 2008,15(29), 3081-94). Since these drugs are the treatment of choice for a multitude of hematologic malignancies and solid tumours, hundred of thousands of patients are affected each year. Sensory abnormalities from antineoplastic-evoked neurotoxicity range from mild paresthesiae or dysesthesiae in many patients, and in some, in chronic painful peripheral neuropathy (Quasthoff and Hartung, J. Neurol., 2002, 249(1), 9-17). The occurrence and severity of the neuropathy is dependent on single dose intensity, duration of treatment, cumulative dose, prior or concurrent treatment with other neuropathic drugs and co-existing conditions such as diabetes and alcohol abuse (Alberts et al., Anticancer Drugs, 1995, 6(3), 369-83; Postma et al., Ann. Oncol., 1995, 6(5), 489-94; Forsyth et al., J. Neurooncol., 1997, 35(1), 47-53; Quasthoff and Hartung, J. Neurol., 2002, 249(1), 9-17). It is known in the art that neuropathic pain, allodynia, hyperalgesia, and especially, peripheral neuropathy, develop in a considerable number of cases as a result of chemotherapy. These are very specific symptoms arising from the neurotoxicity of the chemotherapeutic drug. The treatment of these symptoms is crucial for preserving the quality of life of the afflicted patients (Mielke et al., Eur. J. Cancer, 2006, 42(1), 24-30; Park et al., Curr. Med. Chem., 2008, 15(29), 3081-94; Argyriou et al., Blood, 2008, 112(5), 1593-9). Unfortunately, an effective treatment for chemotherapy-induced peripheral neuropathy has yet to be found (Wolf et al., Eur. J. Cancer, 2008, 44(11), 1507-15).

Therefore, there is a need to provide a new form of treatment and prevention for pain and especially for neuropathic pain, allodynia, hyperalgesia and peripheral neuropathy, developing after chemotherapy.

### BRIEF DESCRIPTION OF THE INVENTION

The inventors of the present invention have surprisingly found and demonstrated that the administration of some specific sigma receptor ligands is highly effective for preventing or treating the pain developing as a consequence of chemotherapy: They are particularly useful when the pain is neuropathic pain, allodynia or hyperalgesia. Even more surprisingly, this invention demonstrates that the co-administration of these sigma ligands and a chemotherapeutic drug prevents the onset of pain frequently associated to chemotherapy.

Therefore, one aspect of the present invention relates to a combination of at least one sigma ligand and at least one chemotherapeutic drug for simultaneous, separate or sequential administration, wherein the sigma ligand has the general formula (I): 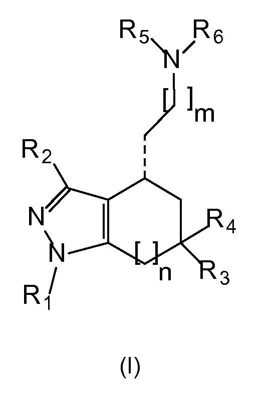 wherein
R₁ is selected from the group formed by hydrogen, susbtituted or unsubstituted alkyl, susbtituted or unsubstituted cycloalkyl, susbtituted or unsubstituted, aromatic or non-aromatic heterocyclyl, susbtituted or unsubstituted aryl, susbtituted or unsubstituted arylalkyl, and susbtituted or unsubstituted heterocyclylalkyl;
R₂ is selected from the group formed by hydrogen, susbtituted or unsubstituted alkyl, susbtituted or unsubstituted cycloalkyl, susbtituted or unsubstituted alkoxy, susbtituted or unsubstituted aryl, susbtituted or unsubstituted, aromatic or non-aromatic heterocyclyl, susbtituted or unsubstituted arylalkyl, and susbtituted or unsubstituted heterocyclylalkyl;
R₃ and R₄ are independently selected from the group formed by hydrogen, susbtituted or unsubstituted alkyl, susbtituted or unsubstituted cycloalkyl, susbtituted or unsubstituted, aromatic or non-aromatic heterocyclyl, susbtituted or unsubstituted aryl, susbtituted or unsubstituted arylalkyl and susbtituted or unsubstituted heterocyclylalkyl or, together, R₃ and R₄ form a 3 to 6 substituted or unsubstituted member ring;
R₅ and R₆ are independently selected from the group formed by hydrogen, susbtituted or unsubstituted alkyl, susbtituted or unsubstituted cycloalkyl, susbtituted or unsubstituted, aromatic or non-aromatic heterocyclyl, susbtituted or unsubstituted aryl, susbtituted or unsubstituted arylalkyl and susbtituted or unsubstituted heterocyclylalkyl or, R₅ and R₆ together, form a substituted or unsubstituted heterocyclyl having 3 to 7 atoms in the ring;
n is selected from 0, 1 and 2;
m is selected from 0, 1, 2, 3 and 4;
the dotted line ----- is either a single or a double bond;
or a pharmaceutically acceptable salt, isomer, prodrug or solvate thereof.

In another aspect, the present invention relates to a compound of formula (I) as defined above, or a pharmaceutically acceptable salt, isomer, prodrug or solvate thereof, for its use in the prevention or treatment of pain induced by chemotherapy.

Another aspect of this invention refers to a combination as defined above for its use in the manufacture of a medicament.

Another aspect of the invention relates to a combination as defined above for its use in the prevention or treatment of pain induced by chemotherapy.

Another aspect of the invention is a method of treatment of a patient suffering from pain induced by chemotherapy, or likely to suffer pain as a result of a chemotherapeutic treatment, which comprises administering to the patient in need of such a treatment or prophylaxis a therapeutically effective amount of a sigma ligand of formula (I) as defined above.

These aspects and preferred embodiments thereof are additionally also defined in the claims.

### BRIEF DESCRIPTION OF THE FIGURES

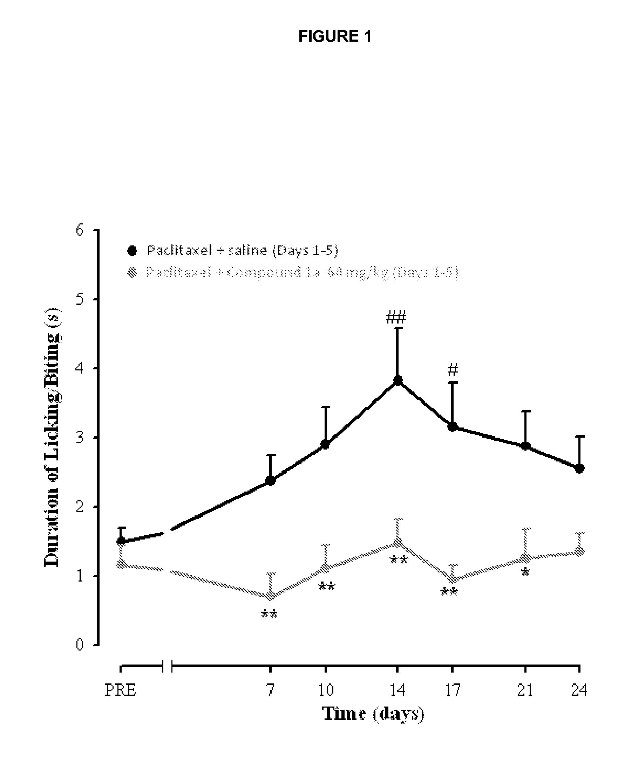

**Fig. 1****:** Time-course of the effect of co-administration of paclitaxel + compound 1a (64 mg/kg) and paclitaxel + saline on duration of hind paw licking/biting in the acetone test. Each point and vertical line represent the mean ± S.E.M. of the values obtained in 16-20 animals per group. Statistically significant differences in comparison to paclitaxel + saline: * p < 0.05, ** ρ < 0.01; and between the values on the pretreatment day and the days after treatment: # *p* < 0.05, ## *p* < 0.01 (two-way repeated measures ANOVA followed by Newman-Keuls test).

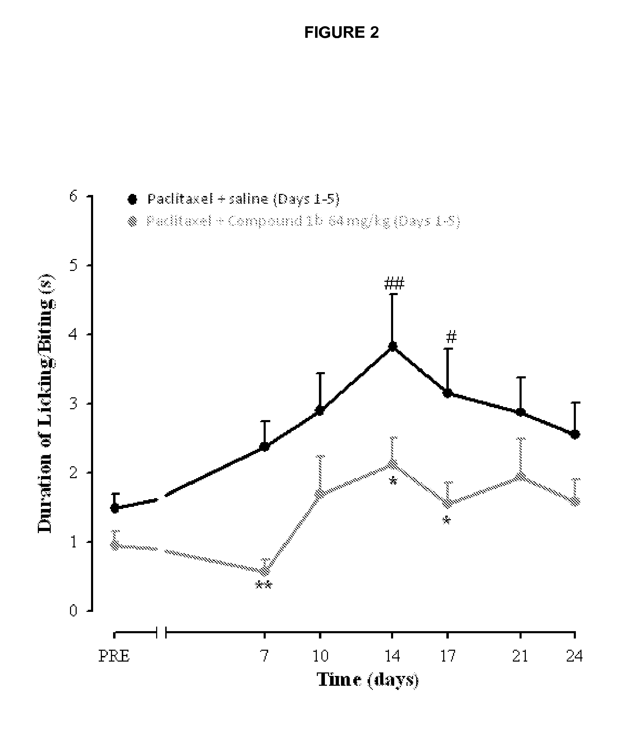

**Fig. 2****:** Time-course of the effect of co-administration of paclitaxel + compound 1b (64 mg/kg) and paclitaxel + saline on duration of hind paw licking/biting in the acetone test. Each point and vertical line represent the mean ± S.E.M. of the values obtained in 16 animals per group. Statistically significant differences in comparison to paclitaxel + saline: * p < 0.05, ** ρ < 0.01; and between the values on the pretreatment day and the days after treatment: # *p* < 0.05, ## *p* < 0.01 (two-way repeated measures ANOVA followed by Newman-Keuls test).

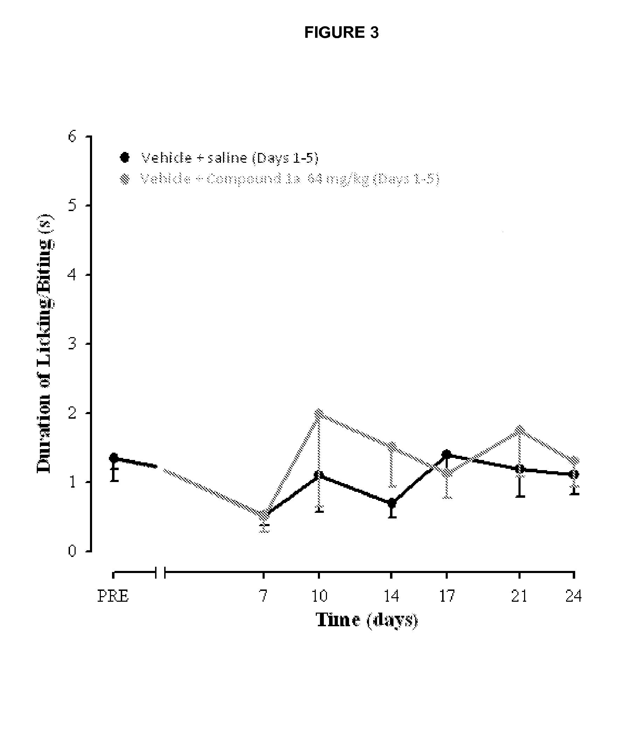

**Fig. 3****:** Time-course of the effect on the duration of hind paw licking/biting (acetone test) of co-administration of paclitaxel vehicle + compound 1 a (64 mg/kg) and paclitaxel vehicle + saline. Each point and vertical line represent the mean ± S.E.M. of the values obtained in 7-9 animals per group. No statistically significant differences between the two groups were observed at any observation time, or in comparison to their own pretreatment day values (two-way repeated measures ANOVA).

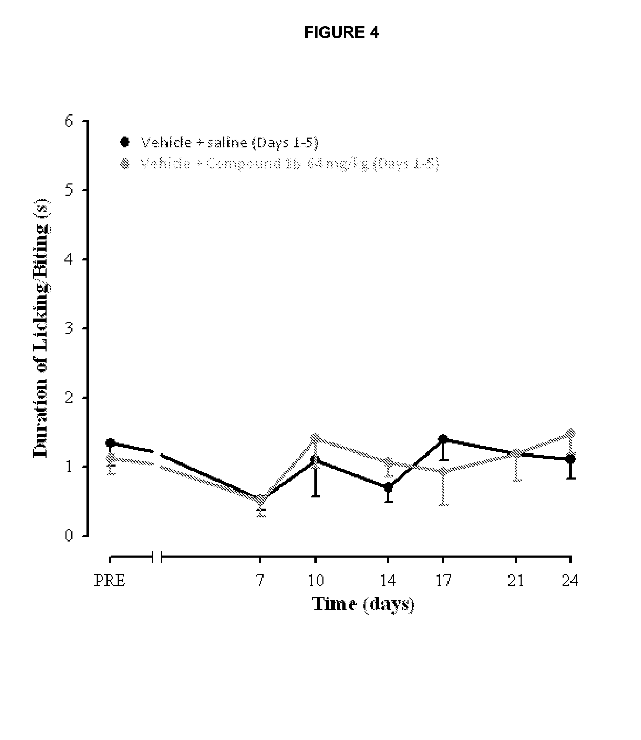

**Fig. 4****:** Time-course of the effect on the duration of hind paw licking/biting (acetone test) of co-administration of paclitaxel vehicle + compound 1 b (64 mg/kg) and paclitaxel vehicle + saline. Each point and vertical line represent the mean ± S.E.M. of the values obtained in 9 animals per group. No statistically significant differences between the two groups were observed at any observation time, or in comparison to their own pretreatment day values (two-way repeated measures ANOVA).

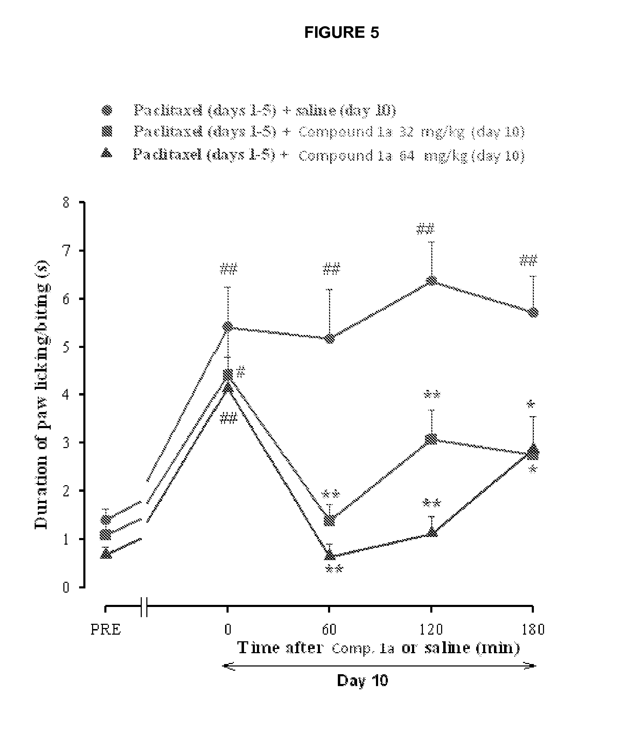

**Fig. 5****:** Time-course of the effect on the duration of hind paw licking/biting (acetone test) of a single s.c. injection of compound 1a (32 or 64 mg/kg) or saline in mice pretreated with paclitaxel. Each point and vertical line represent the mean ± S.E.M. of the values obtained in 14-22 animals per group. Statistically significant differences between the compound 1 a- and saline-treated groups on the same day after treatment: * p < 0.05; ** ρ < 0.01; and between the values obtained in the pretreatment day and in day 10 at different times after drug or saline administration: # *p* < 0.05; ## *p* < 0.01 (two-way repeated measures ANOVA followed by Newman-Keuls test).

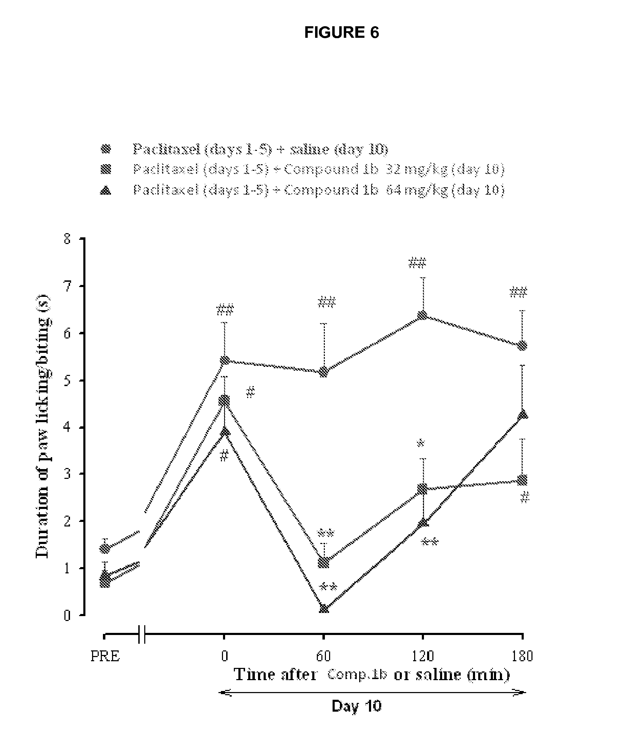

**Fig. 6****:** Time-course of the effect on the duration of hind paw licking/biting (acetone test) of a single s.c. injection of compound 1 b (32 or 64 mg/kg) or saline in mice pretreated with paclitaxel. Each point and vertical line represent the mean ± S.E.M. of the values obtained in 14-22 animals per group. Statistically significant differences between the compound 1 b- and saline-treated groups on the same day after treatment: * p < 0.05; ** ρ < 0.01; and between the values obtained in the pretreatment day and in day 10 at different times after drug or saline administration: # *p* < 0.05; ## *p* < 0.01 (two-way repeated measures ANOVA followed by Newman-Keuls test).

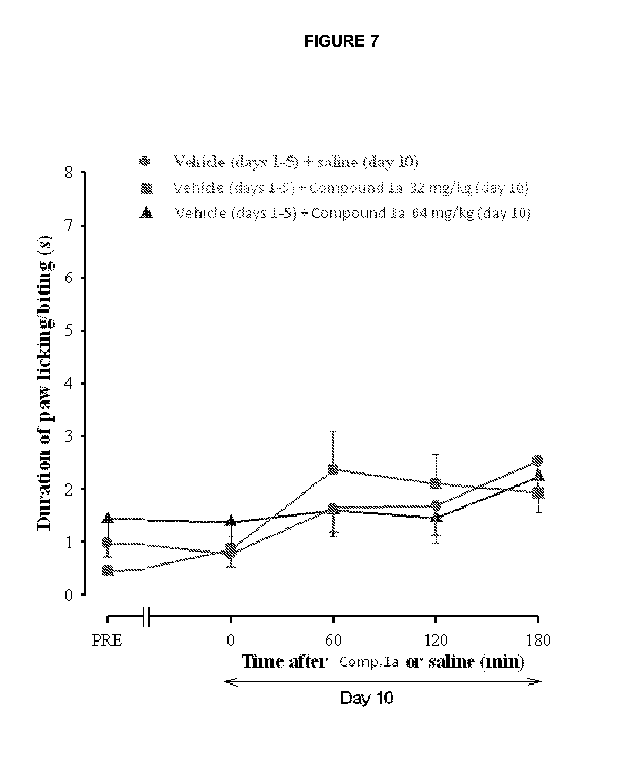

**Fig. 7****:** Time-course of the effect on the duration of hind paw licking/biting (acetone test) of a single s.c. injection of compound 1 a (64 mg/kg) or saline in mice pretreated with paclitaxel-vehicle. Each point and vertical line represent the mean ± S.E.M. of the values obtained in 8-14 animals per group. No statistically significant differences were found between the two groups at any observation time, or in comparison to their own pretreatment day values (two-way repeated measures ANOVA).

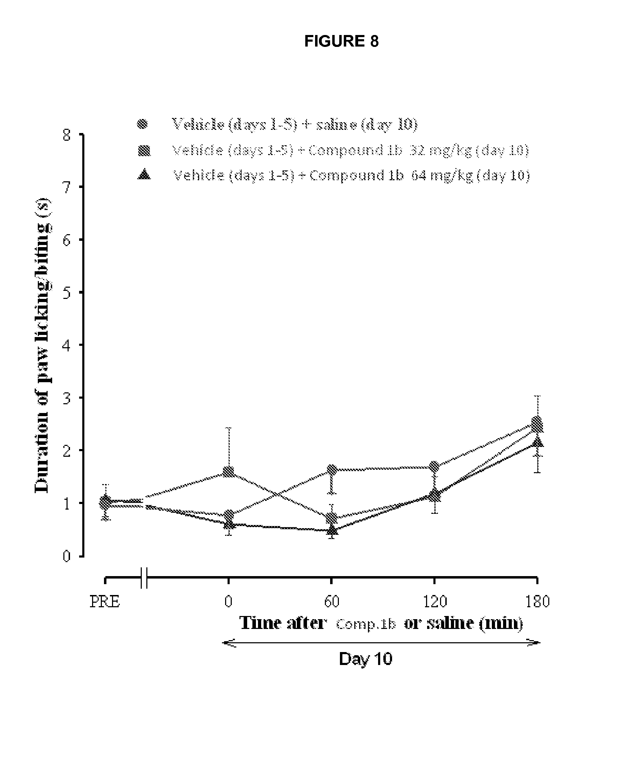

**Fig. 8****:** Time-course of the effect on the duration of hind paw licking/biting (acetone test) of a single s.c. injection of compound 1b (64 mg/kg) or saline in mice pretreated with paclitaxel-vehicle. Each point and vertical line represent the mean ± S.E.M. of the values obtained in 8-14 animals per group. No statistically significant differences were found between the two groups at any observation time, or in comparison to their own pretreatment day values (two-way repeated measures ANOVA).

### DETAILED DESCRIPTION OF THE INVENTION

In the context of the present invention, the following terms have the meaning detailed below.

"Alkyl" refers to a straight or branched hydrocarbon chain radical consisting of 1 to 12 carbon and hydrogen atoms, containing no unsaturation, preferably having 1 to 6 carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as aryl, halo, hydroxy, alkoxy, carboxy, cyano, carbonyl, acyl, alkoxycarbonyl, amino, nitro, mercapto, alkylthio, etc. If substituted by aryl it corresponds to an "Arylalkyl" radical, such as benzyl and phenethyl. Preferred alkyl radicals have from 1-6 carbon atoms.

"Alkenyl" refers to an alkyl radical consisting of 2 to 12 carbons and having one or more unsaturated bonds.

"Cycloalkyl" refers to a stable 3-to 10-membered monocyclic or bicyclic radical which is saturated or partially saturated, and which consists solely of carbon and hydrogen atoms, such as cyclohexyl or adamantyl. Unless otherwise stated specifically in the specification, the term"cycloalkyl" is meant to include cycloalkyl radicals which are optionally substituted by one or more substituents such as alkyl, halo, hydroxy, amino, cyano, nitro, alkoxy, carboxy, alkoxycarbonyl, etc.

"Aryl" refers to single and multiple aromatic ring radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, phenanthryl or anthryl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

"Heterocyclyl" refers to a stable 3-to 15 membered ring radical, aromatic or not, which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be aromatic, saturated or partially saturated, and also a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc. Unless otherwise stated specifically in the specification, the term "heterocyclyl" is meant to include heterocyclyl radicals which are optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, sulfonyl, sulfonylamino, alkyl or arylsulfonylamino, alkylarylsulfonilamino, acylamino, acetamido, etc.

"Alkoxy" refers to a radical of the formula -ORa where Ra is an alkyl radical as defined above, e. g., methoxy, ethoxy, propoxy, etc.

"Alkoxycarbonyl" refers to a radical of the formula -C(O)ORa where Ra is an alkyl radical as defined above, e. g., methoxycarbonyl, ethoxycarbonyl, propoxycarbonyl, etc.

"Alkylthio" refers to a radical of the formula-SRa where Ra is an alkyl radical as defined above, e. g., methylthio, ethylthio, propylthio, etc.

"Amino" refers to a radical of the formula-NH₂, -NHRa or -NRaRb, optionally quaternized, wherein Ra and Rb are, independently, an alkyl radical as defined above, e.g., methylamino, ethylamino, dimethylamino, diethylamino, propylamino, etc.

"Halo" or "hal" refers to bromo, chloro, iodo or fluoro.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C1-6 alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocyclic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "salt" must be understood as any form of an active compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used on humans and/or mammals. Salts with alkali and alkali earth metals are preferred particularly, as well as those formed with ammonium cations (NH₄⁺). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of a compound of formula (I) is also within the scope of the invention. The term "prodrug" is used in its broadest sense and encompasses those derivatives that are converted in vivo to the compounds of the invention. Examples of prodrugs include, but are not limited to, derivatives and metabolites of the compounds of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

As used herein, the terms "treat", "treating" and "treatment" include the eradication, removal, reversion, alleviation, modification, or control of pain induced by chemotherapy, after the pain onset.

As used herein, the terms "prevention", "preventing", "preventive" "prevent" and "prophylaxis" refers to the capacity of a therapeutic to avoid, minimize or difficult the onset or development of a disease or condition before its onset, in this case pain induced by chemotherapy.

As used herein the term "chemotherapy" or "chemotherapeutic drug" refers broadly to the use of a chemical drugs for the treatment of cancer, tumors or malign neoplasia. Examples of chemical drugs used to treat cancer are cytostatic and cytotoxic drugs, but it is not limited only to these.

"Developing as a consequence of chemotherapy" according to this invention is defined as: a) developing after or with the beginning of chemotherapy and b) thus coinciding with or following the use of a chemotherapeutic drug. Therefore, the symptom to be treated is likely to be caused by or is due to the toxicity, cytotoxicity or especially, the peripheral neurotoxicity, of the chemotherapeutic drug.

In a preferred embodiment, R₁ in compounds of formula (I) is selected from the group consisting of susbtituted or unsubstituted alkyl, susbtituted or unsubstituted cycloalkyl, susbtituted or unsubstituted, aromatic or non-aromatic heterocyclyl and susbtituted or unsubstituted aryl; more preferably susbtituted or unsubstituted alkyl, unsubstituted cycloalkyl, unsubstituted aryl and aromatic heterocyclic; and most preferred, methyl, t-butyl, cyclohexyl and phenyl.

In another preferred embodiment, R₂ represents hydrogen or alkyl; more preferably hydrogen.

In yet another preferred embodiment of the invention, R₃ and R₄ are both hydrogen or alkyl; more preferably both are hydrogen or methyl; and most preferably both are hydrogen.

Also, embodiments where m is 1 or 2 are preferred in the context of the present invention, more preferably m is 1. Moreover, it is also preferred that n is 0 or 1, more preferably it is 1.

In another preferred embodiment, the dotted line is a single bond.

Finally, in another embodiment it is preferred that R₅ and R₆ together form a substituted or unsubstituted heterocyclyl having 3 to 7 atoms in the ring, in particular morpholin-4-yl, 2,6-dimethylmorpholin-4-yl, piperidin-1-yl, 4-phenylpiperidin-1-yl, 3-phenylpiperidin-1-yl, 4-benzylpiperazin-1-yl, 4-phenyl-piperazin-1-yl, 2-[spiro [isobenzofuran-1(3H),4'-piperidin]-1'-yl, azepan-1-yl, 1,2,3,4-tetrahidro-isoquinolin-2-yl, pyrrolidin-1-yl, 3-phenylpyrrolidin-1-yl, isoindolin-2-yl or imidazol-1-yl. Preferably, R₅ and R₆ together form a morpholin-4-yl group.

In preferred variants of the invention, the sigma ligand of formula (I) is selected from:

| | |
|---|---|
| 1. | 4,5,6,7-tetrahydro-4-(2-(morpholin-4-yl)ethyl)-1-phenyl-1H-indazole, |
| 2. | 4,5,6,7-tetrahydro-4-(2-(morpholin-4-yl)ethyl)-1-phenyl-1H-indazole oxalate, |
| 3. | 1-(3,4-dichlorophenyl)-4,5,6,7-tetrahydro-4-(2-(4-phenylpiperidin-1-yl)ethyl)-1H-indazole, |
| 4. | 1-(3,4-dichlorophenyl)-4,5,6,7-tetrahydro-4-(2-(4-phenylpiperidin-1-yl)ethyl)-1H-indazole oxalate, |
| 5. | (E)-4,5,6,7-tetrahydro-4-(2-(morpholin-4-yl)ethylidene)-1-phenyl-1H-indazole, |
| 6. | 1-(3,4-dichlorophenyl)-4,5,6,7-tetrahydro-4-(2-(morpholin-4-yl)ethyl)-1H-indazole, |
| 7. | 1-(3,4-dichlorophenyl)-4,5,6,7-tetrahydro-4-(2-(morpholin-4-yl)ethyl)-1H-indazole oxalate, |
| 8. | 4,5,6,7-tetrahydro-1-phenyl-4-(2-(4-phenylpiperidin-1-yl)ethyl)-1H-indazole, |
| 9. | 4,5,6,7-tetrahydro-1-phenyl-4-(2-(4-phenylpiperidin-1-yl)ethyl)-1H-indazole oxalate, |
| 10. | 4-(2-(4-benzylpiperazin-1-yl)ethyl)-4,5,6,7-tetrahydro-1-phenyl-1H-indazole, |
| 11. | 4,5,6,7-tetrahydro-1-phenyl-4-(2-[spiro[isobenzofuran-1(3H),4'-piperidin]-1'-yl] ethyl)-1H-indazole, |
| 12. | 4,5,6,7-tetrahydro-1-phenyl-4-(2-[spiro[isobenzofuran-1(3H),4'-piperidin]-1'-yl] ethyl)-1H-indazole oxalate, |
| 13. | 4,5,6,7-tetrahydro-1-phenyl-4-(2-(piperidin-1-yl)ethyl)-1H-indazole, |
| 14. | 4,5,6,7-tetrahydro-1-phenyl-4-(2-(piperidin-1-yl)ethyl)-1H-indazoleoxalate, |
| 15. | (E)-4-(2-(azepan-1-yl)ethylidene)-4,5,6,7-tetrahydro-1-phenyl-1H-indazole, |
| 16. | (E)-4-(2-(azepan-1-yl)ethylidene)-4,5,6,7-tetrahydro-1-phenyl-1H-indazole oxalate, |
| 17. | (E)-4,5,6,7-tetrahydro-1-phenyl-4-(2-(4-phenylpiperidin-1-yl)ethylidene)-1H-indazole, |
| 18. | (E)-4,5,6,7-tetrahydro-1-phenyl-4-(2-[spiro[isobenzofuran-1(3H),4'-piperidin]-1'-yl] ethylidene)-1H-indazole, |
| 19. | (E)-4,5,6,7-tetrahydro-1-phenyl-4-(2-[spiro[isobenzofuran-1(3H),4'-piperidin]-1'-yl] ethylidene)-1H-indazole oxalate, |
| 20. | (E)-4,5,6,7-tetrahydro-1-phenyl-4-(2-(4-phenylpiperazin-1-yl)ethylidene)-1H-indazole, |
| 21. | 1,2,3,4-tetrahydro-2-((E)-2-(6,7-dihydro-1-phenyl-1H-indazol-4(5H)-ylidene)ethyl) isoquinoline, |
| 22. | 1,2,3,4-tetrahydro-2-((E)-2-(6,7-dihydro-1-phenyl-1H-indazol-4(5H)-ylidene)ethyl) isoquinoline oxalate, |
| 23. | (E)-4,5,6,7-tetrahydro-1-phenyl-4-(2-(3-phenylpiperidin-1-yl)ethylidene)-1H-indazole, |
| 24. | (E)-4,5,6,7-tetrahydro-1-phenyl-4-(2-(3-phenylpyrrolidin-1-yl)ethylidene)-1H-indazole |
| 25. | (E)-4,5,6,7-tetrahydro-1-phenyl-4-(2-(3-phenylpyrrolidin-1-yl)ethylidene)-1H-indazole oxalate, |
| 26. | (E)-4,5,6,7-tetrahydro-4-(2-(isoindolin-2-yl)ethylidene)-1-phenyl-1H-indazole, |
| 27. | (E)-4,5,6,7-tetrahydro-1-phenyl-4-(2-(piperidin-1-yl)ethylidene)-1H-indazole, |
| 28. | 1-(3,4-dichlorophenyl)-4,5,6,7-tetrahydro-4-(2-(4-phenylpiperazin-1-yl)ethyl)-1H-indazole, |
| 29. | 1-(3,4-dichlorophenyl)-4,5,6,7-tetrahydro-4-(2-(4-phenylpiperazin-1-yl)ethyl)-1H-indazole oxalate, |
| 30. | 1,4,5,6-tetrahydro-4-(2-(morpholin-4-yl)ethyl)-1-phenylcyclopenta[c]pyrazole, |
| 31. | 1,4,5,6-tetrahydro-4-(2-(morpholin-4-yl)ethyl)-1-phenylcyclopenta[c]pyrazole oxalate, |
| 32. | 4,5,6,7-tetrahydro-1-phenyl-4-(2-(4-phenylpiperazin-1-yl)ethyl)-1H-indazole, |
| 33. | 4,5,6,7-tetrahydro-1-phenyl-4-(2-(4-phenylpiperazin-1-yl)ethyl)-1H-indazole oxalate, |
| 34. | 2-(2-(1,4,5,6-tetrahydro-1-phenylcyclopenta[c]pyrazol-4-yl)ethyl)isoindoline, |
| 35. | 2-(2-(1,4,5,6-tetrahydro-1-phenylcyclopenta[c]pyrazol-4-yl)ethyl)isoindoline oxalate, |
| 36. | 1,4,5,6-tetrahydro-1-phenyl-4-(2-(4-phenylpiperidin-1-yl)ethyl)cyclopenta[c] pyrazole, |
| 37. | 1,4,5,6-tetrahydro-1-phenyl-4-(2-(piperidin-1-yl)ethyl)cyclopenta[c]pyrazole, |
| 38. | 1,4,5,6-tetrahydro-1-phenyl-4-(2-(4-phenylpiperazin-1-yl)ethyl)cyclopenta[c] pyrazole, |
| 39. | 1,4,5,6-tetrahydro-1-phenyl-4-(2-(pyrrolidin-1-yl)ethyl)cyclopenta[c]pyrazole, |
| 40. | 1,4,5,6-tetrahydro-1-phenyl-4-(2-(pyrrolidin-1-yl)ethyl)cyclopenta[c]pyrazole oxalate, |
| 41. | 4-(2-(4-benzylpiperazin-1-yl)ethyl)-1,4,5,6-tetrahyd ro-1-phenylcyclopenta[c] pyrazole, |
| 42. | 4-(2-(4-benzylpiperazin-1-yl)ethyl)-1,4,5,6-tetrahydro-1-phenylcyclopenta[c] pyrazole dioxalate, |
| 43. | 1,2,3,4-tetrahydro-2-(2-(1,4,5,6-tetrahydro-1-phenylcyclopenta[c]pyrazol-4-yl)ethyl) isoquinoline, |
| 44. | 1,2,3,4-tetrahydro-2-(2-(1,4,5,6-tetrahydro-1-phenylcyclopenta[c]pyrazol-4-yl)ethyl) isoquinoline oxalate, |
| 45. | 4-(2-(1H-imidazol-1-yl)ethyl)-1,4,5,6-tetrahyd ro-1-phenylcyclopenta[c]pyrazole, |
| 46. | *cis*-14,5,6-tetrahydro-4-(2-(2,6-dimethylmorpholin-4-yl)ethyl)-1-phenylcyclopenta [c]pyrazole, |
| 47. | *cis*-14,5,6-tetrahydro-4-(2-(2,6-dimethylmorpholin-4-yl)ethyl)-1-phenylcyclopenta [c]pyrazole oxalate, |
| 48. | *cis*-4,5,6,7-tetrahydro-4-(2-(2,6-dimethylmorpholin-4-yl)ethyl)-1-phenyl-1H-indazole, |
| 49. | *cis*-4,5,6,7-tetrahydro-4-(2-(2,6-dimethylmorpholin-4-yl)ethyl)-1-phenyl-1H-indazole oxalate, |
| 50. | 1,4,5,6-tetrahydro-1-phenyl-4-(2-[spiro[isobenzofuran-1(3H),4'-piperidin]-1'-yl]-ethyl)cyclopenta[c]pyrazole, |
| 51. | 1,4,5,6-tetrahydro-1-phenyl-4-(2-[spiro[isobenzofuran-1(3H),4'-piperidin]-1'-yl]-ethyl)cyclopenta[c]pyrazole oxalate, |
| 52. | N-benzyl-2-(1,4,5,6-tetrahydro-1-phenylcyclopenta[c]pyrazol-4-yl)-N-methyl-ethanamine, |
| 53. | diastereomeric mixture of 1,4,5,6-tetrahydro-1-phenyl-4-(2-(3-phenylpiperidin-1-yl)ethyl)cyclopenta[c]pyrazole, |
| 54. | N-benzyl-2-(4,5,6,7-tetrahydro-1-phenyl-1H-indazol-4-yl)-N-methylethanamine, |
| 55. | 4,5,6,7-tetrahydro-4-(2-(isoindolin-2-yl)ethyl)-1-phenyl-1H-indazole, |
| 56. | 4,5,6,7-tetrahydro-4-(2-(isoindolin-2-yl)ethyl)-1-phenyl-1H-indazoleoxalate, |
| 57. | 4,5,6,7-tetrahydro-1-methyl-4-(2-(4-phenylpiperidin-1-yl)ethyl)-1H-indazole, |
| 58. | 4,5,6,7-tetrahydro-1-methyl-4-(2-(4-phenylpiperidin-1-yl)ethyl)-1H-indazole oxalate, |
| 59. | 4,5,6,7-tetrahydro-4-(2-(isoindolin-2-yl)ethyl)-1-methyl-1H-indazole, |
| 60. | 4,5,6,7-tetrahydro-4-(2-(isoindolin-2-yl)ethyl)-1-methyl-1H-indazole oxalate, |
| 61. | N-benzyl-2-(4,5,6,7-tetrahydro-1-methyl-1H-indazol-4-yl)-N-methylethanamine, |
| 62. | N-benzyl-2-(4,5,6,7-tetrahydro-1-methyl-1H-indazol-4-yl)-N-methylethanamine oxalate, |
| 63. | 4,5,6,7-tetrahydro-1-methyl-4-(2-(morpholin-4-yl)ethyl)-1H-indazole, |
| 64. | 4,5,6,7-tetrahydro-1-methyl-4-(2-(morpholin-4-yl)ethyl)-1H-indazole oxalate, |
| 65. | 1-(4-fluorophenyl)-4,5,6,7-tetrahydro-4-(2-(morpholin-4-yl)ethyl)-1H-indazole, |
| 66. | 1-(4-fluorophenyl)-4,5,6,7-tetrahydro-4-(2-(morpholin-4-yl)ethyl)-1H-indazole oxalate, |
| 67. | *cis*-1-(4-fluorophenyl)-4,5,6,7-tetrahydro-4-(2-(2,6-dimethylmorpholin-4-yl)ethyl)-1H-indazole, |
| 68. | *cis*-1-(4-fluorophenyl)-4,5,6,7-tetrahydro-4-(2-(2,6-dimethylmorpholin-4-yl)ethyl)-1H-indazole oxalate, |
| 69. | N-benzyl-2-(1-(4-fluorophenyl)-4,5,6,7-tetrahydro-1H-indazol-4-yl)-N-methyl-ethanamine, |
| 70. | 1-(4-fluorophenyl)-4,5,6,7-tetrahydro-4-(2-(isoindolin-2-yl)ethyl)-1H-indazole, |
| 71. | 1-(4-fluorophenyl)-4,5,6,7-tetrahydro-4-(2-(isoindolin-2-yl)ethyl)-1H-indazole oxalate, |
| 72. | 1-(4-fluorophenyl)-4,5,6,7-tetrahydro-4-(2-(4-phenylpiperidin-1-yl)ethyl)-1H-indazole, |
| 73. | (+)-4,5,6,7-tetrahydro-4-(2-(morpholin-4-yl)ethyl)-1-phenyl-1H-indazole, |
| 74. | (+)-4,5,6,7-tetrahydro-4-(2-(morpholin-4-yl)ethyl)-1-phenyl-1H-indazoleoxalate, |
| 75. | (-)-4,5,6,7-tetrahydro-4-(2-(morpholin-4-yl)ethyl)-1-phenyl-1H-indazole, |
| 76. | (-)-4,5,6,7-tetrahydro-4-(2-(morpholin-4-yl)ethyl)-1-phenyl-1H-indazoleoxalate, |
| 77. | (E)-4,5,6,7-tetrahydro-4-(2-(morpholin-4-yl)ethylidene)-1-phenyl-1H-indazole oxalate |
| 78. | (E)-4,5,6,7-tetrahydro-1-phenyl-4-(2-(piperidin-1-yl)ethylidene)-1H-indazole oxalate, |
| 79. | (E)-N-benzyl-2-(6,7-dihydro-1-phenyl-1H-indazol-4(5H)-ylidene)-N-methyl-ethanamine, |
| 80. | (E)-N-benzyl-2-(6,7-dihydro-1-phenyl-1H-indazol-4(5H)-ylidene)-N-methyl-ethanamine oxalate, |
| 81. | (E)-4,5,6,7-tetrahydro-4-(2-(*cis*-2,6-dimethylmorpholin-4-yl)ethylidene)-1-phenyl-1H-indazole, |
| 82. | (E)-4,5,6,7-tetrahydro-4-(2-(*cis*-2,6-dimethylmorpholin-4-yl)ethylidene)-1-phenyl-1H-indazole oxalate, |
| 83. | (E)-1-(3,4-dichlorophenyl)-4,5,6,7-tetrahydro-4-(2-(*cis*-2,6-dimethylmorpholino-4-yl)-ethylidene)-1H-indazole |
| 84. | (E)-1-(3,4-dichlorophenyl)-4,5,6,7-tetrahydro-4-(2-(*cis*-2,6-dimethyl- morpholino-4-yl)ethylidene)-1H-indazole oxalate |
| 85. | (E)-1-(3,4-dichlorophenyl)-4,5,6,7-tetrahydro-4-(2-(morpholino-4-yl)ethylidene)-1H-indazole |
| 86. | (E)-1-(3,4-dichlorophenyl)-4,5,6,7-tetrahydro-4-(2-(morpholino-4-yl)-ethylidene)-1H-indazole oxalate |
| 87. | (E)-1-(3,4-dichlorophenyl)-4,5,6,7-tetrahydro-4-(2-(piperidin-1-yl)ethylidene)-1H-indazole |
| 88. | (E)-1-(3,4-dichlorophenyl)-4,5,6,7-tetrahydro-4-(2-(piperidin-1-yl)-ethylidene)-1H-indazole oxalate |
| 89. | (E)-4-(2-(N,N-diethylamino)ethylidene)- 4,5,6,7-tetrahydro-1-phenyl-1H-indazole |
| 90. | (E)-4-(2-(N,N-diethylamino)ethylidene)- 4,5,6,7-tetrahydro-1-phenyl-1H-indazole oxalate |
| 91. | (E)-4-(2-(4-cyclohexylpiperazin-1-yl)ethylidene)-4,5,6,7-tetrahydro-1-phenyl-1H-indazole |
| 92. | (E)-4,5,6,7-tetrahydro-4-(2-(4-methylpiperidin-1-yl)ethylidene)-1-phenyl-1H-indazole |
| 93. | 4,5,6,7-tetrahydro-4-(2-(4-methylpiperidin-1-yl)ethyl)-1-phenyl-1H-indazole |
| 94. | 4,5,6,7-tetrahydro-4-(2-(4-methylpiperidin-1-yl)ethyl)-1-phenyl-1H-indazole oxalate |
| 95. | 1,4,5,6-tetrahydro-1-phenyl-4-(2-(piperidin-1-yl)ethyl)cyclopenta[c]pyrazole oxalate |
| 96. | 1,4,5,6-tetrahydro-1-phenyl-4-[2-(thiomorpholin-4-yl)ethyl]cyclopenta[c]pyrazole |
| 97. | 1,4,5,6-tetrahydro-1-phenyl-4-[2-(thiomorpholin-4-yl)ethyl]cyclopenta[c]pyrazole oxalate |
| 98. | 1,4,5,6-tetrahydro-4-(2-(4-methylpiperidin-1-yl)ethyl)-1-phenylcyclopenta[c]pyrazole |
| 99. | 1,4,5,6-tetrahydro-4-(2-(4-methylpiperidin-1-yl)ethyl)-1-phenylcyclopenta[c]pyrazole oxalate |
| 100. | 1,4,5,6-tetrahydro-4-(2-(4-methylpiperazin-1-yl)ethyl)-1-phenylcyclopenta[c] pyrazole |
| 101. | 4-(2-(4-cyclohexylpiperazin-1-yl)ethyl)-1,4,5,6-tetrahydro-1-phenylcyclopenta[c] pyrazole |
| 102. | 4-(2-(azepan-1-yl)ethyl)-4,5,6,7-tetrahydro-1-phenyl-1H-indazole |
| 103. | N-benzyl-2-(1-tert-butyl-4,5,6,7-tetrahydro-1H-indazol-4-yl)-N-methylethanamine |
| 104. | 1-tert-butyl-4,5,6,7-tetrahydro-4-(2-(4-phenylpiperidin-1-yl)ethyl)-1H-indazole |
| 105. | 1-tert-butyl-4,5,6,7-tetrahydro-4-(2-(4-phenylpiperidin-1-yl)ethyl)-1H-indazole oxalate |
| 106. | (-)-N-bencyl-2-(4,5,6,7-tetrahydro-1-methyl-1H-indazol-4-yl)-N-methylethanamine |
| 107. | (+)-N-bencyl-2-(4,5,6,7-tetrahydro-1-methyl-1H-indazol-4-yl)-N-methylethanamine |
| 108. | N-(2-(4,5,6,7-tetrahydro-1-methyl-1H-indazol-4-yl)ethyl)-N-methylcyclo hexanamine |
| 109. | 4,5,6,7-tetrahydro-4-(2-(4-hydroxy-4-phenylpiperidin-1-yl)ethyl)-1-methyl-1H-indazole |
| 110. | 4,5,6,7-tetrahydro-1-methyl-4-(2-(4-phenylpiperidin-1-yl)ethyl)-1H-indazole |
| 111. | N-benzyl-2-(4,5,6,7-tetrahydro-1-methyl-1H-indazol-4-yl)-N-methylethanamine |
| 112. | 4,5,6,7-tetrahydro-1-phenyl-4-(2-[spiro[isobenzofuran-1(3H),4'-piperidin]-1'-yl]-ethyl)-1H-indazole |

or their pharmaceutically acceptable salts, estereoisomers, tautomers, solvates or a prodrug thereof.

In a more preferred variant, the sigma ligand of formula (I) is selected from (S)-4,5,6,7-tetrahydro-4-(2-(morpholin-4-yl)ethyl)-1-phenyl-1H-indazole hydrochloride and (R)- 4,5,6,7-tetrahydro-4-(2-(morpholin-4-yl)ethyl)-1-phenyl-1H-indazole hydrochloride. These compounds are designated in the examples of the present invention as compound **1a** and compound **1b**, respectively.

Other salts of compounds **1a** and **1b** are also preferred.

Any compound referred to herein is intended to represent such specific compound as well as certain variations or forms. In particular, compounds referred to herein may have asymmetric centres and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same as, or different to, the stereoisomerism of the other double bonds of the molecule. Furthermore, compounds referred to herein may exist as atropisomers. All the stereoisomers including enantiomers, diastereoisomers, geometric isomers and atropisomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by 13C- or 14C-enriched carbon, or the replacement of at least one nitrogen by 15N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of formula (I) can be prepared as disclosed in the previous application WO2006/021463.

In a preferred embodiment of the invention, the pain is induced by a chemotherapeutic drug selected from drugs derived from platinum, plant alkaloids and terpenes (terpenoids).

More preferably the drugs derived from platinum are the commercially available cisplatin, carboplatin and oxaliplatin.

"Plant alkaloids" (and terpenoids) are alkaloids derived from plants that block cell division by preventing microtubule function. Since microtubules are vital for cell division, their inhibition also arrests cell mitosis. The main examples of plant alkaloids are vinca alkaloids and taxanes.

"Vinca alkaloids" bind to specific sites on tubulin, inhibiting the assembly of tubulin into microtubules (M phase of the cell cycle). They are derived from the Madagascar periwinkle, *Catharanthus roseus* (formerly known as *Vinca rosea*). The preferred vinca alkaloids include Vincristine, Vinblastine, Vinorelbine, and Vindesine.

"Taxanes" are derived from the Pacific yew tree, *Taxus brevifolia.* Taxanes enhance the stability of microtubules, preventing the separation of chromosomes during anaphase. Preferred taxanes include Paclitaxel and Docetaxel.

Examples of chemotherapeutic drugs (by their trademarks) that can induce pain that can be prevented or treated with the sigma ligands of formula (I) are:
13-cis-Retinoic Acid, 2-CdA, 2-Chlorodeoxyadenosine, 5-fluorouracil 5-FU, 6-Mercaptopurine, 6-MP, 6-TG 6-Thioguanine, Abraxane, Accutane ®, Actinomycin-D, Adriamycin ®, Adrucil ®, Agrylin ®, Ala-Cort ®, Aldesleukin, Alemtuzumab, ALIMTA, Alitretinoin, Alkaban-AQ ®, Alkeran ®, All-transretinoic acid, Alpha interferon, Altretamine, Amethopterin, Amifostine, Aminoglutethimide, Anagrelide, Anandron ®, Anastrozole, Arabinosylcytosine, Ara-C, Aranesp ®, Aredia ®, Arimidex ®, Aromasin ®, Arranon ®, Arsenic trioxide, Asparaginase, ATRA, Avastin ®, Azacitidine"BCG, BCNU, Bevacizumab, Bexarotene, BEXXAR ®, Bicalutamide, BiCNU, Blenoxane ®, Bleomycin, Bortezomib, Busulfan, Busulfex ®"C225, Calcium Leucovorin, Campath ®, Camptosar ®, Camptothecin-11, Capecitabine, Carac TM, Carboplatin, Carmustine, Carmustine wafer, Casodex ®, CC-5013, CCNU (o), CDDP (t), CeeNU (t), Cerubidine (t), cetuximab, Chlorambucil, Cisplatin, Citrovorum Factor, Cladribine, Cortisone, Cosmegen (t), CPT-11 (o), Cyclophosphamide, Cytadren (t), Cytarabine, Cytarabine liposomal, Cytosar-U (t), Cytoxan ®, Dacarbazine, Dactinomycin, Darbepoetin alfa, Daunomycin, Daunorubicin, Daunorubicin hydrochloride (t), Daunorubicin liposomal,
DaunoXome (t), Decadron, Delta-Cortef (t), Deltasone (t), Denileukin, diftitox, DepoCyt (t), Dexamethasone, Dexamethasone acetate, dexamethasone sodium phosphate,
Dexasone (t), Dexrazoxane, DHAD (o), DIC (t), Diodex (t), Docetaxel, Doxil (t), Doxorubicin, Doxorubicin liposomal, Droxia (t), DTIC, DTIC-Dome (t), Duralone (t),
Efudex (t), Eligard (t), Ellence (t), Eloxatin (t), Elspar (t), Emcyt (t), Epirubicin, Epoetin alfa, Erbitux, Erlotinib, Erwinia L-asparaginase (t), Estramustine, Ethyol, Etopophos (t), Etoposide, Etoposide phosphate (t), Eulexin (t), Evista (t), Exemestane, Fareston (t),
Faslodex (t), Femara ®, Filgrastim, Floxuridine, Fludara (t), Fludarabine, Fluoroplex (t), Fluorouracil, Fluorouracil (cream), Fluoxymesterone, Flutamide, Folinic Acid (o), FUDR (t), Fulvestrant, G-CSF (t), Gefitinib, Gemcitabine, Gemtuzumab ozogamicin, Gemzar (t), Gleevec™, Gliadel wafer (t), GM-CSF (o), Goserelin, granulocyte - colony stimulating factor (t), Granulocyte macrophage colony stimulating factor (o), Halotestin (t), Herceptin (t), Hexadrol (t), Hexalen (t), Hexamethylmelamine (t), HMM (t), Hycamtin (t), Hydrea (t), Hydrocort Acetate (t), Hydrocortisone, Hydrocortisone sodium phosphate, Hydrocortisone sodium succinate, Hydrocortone phosphate (t), Hydroxyurea, Ibritumomab, Ibritumomab Tiuxetan, Idamycin ®, Idarubicin Ifex ®, IFN-alpha, Ifosfamide, IL-11, IL-2, Imatinib mesylate, Imidazole Carboxamide, Interferon alfa, Interferon Alfa-2b (PEG conjugate) (o), Interleukin - 2 (t), Interleukin-11 (o), Intron A@ (interferon alfa-2b), Iressa ®, Irinotecan, Isotretinoin, Kidrolase (t), Lanacort (t), L-asparaginase (t), LCR (o), Lenalidomide (Lenolidamide), Letrozole, Leucovorin,
Leukeran (t), Leukine (t), Leuprolide, Leurocristine (o), Leustatin (t), Liposomal Ara-C (t), Liquid Pred (t), Lomustine, L-PAM (o), L-Sarcolysin (o), Lupron (t), Lupron Depot (t),
Matulane (t), Maxidex (t), Mechlorethamine, Mechlorethamine Hydrochloride,
Medralone (t), Medrol ®, Megace (t), Megestrol, Megestrol Acetate (o), Melphalan, Mercaptopurine, Mesna, Mesnex (t), Methotrexate, Methotrexate Sodium (o), Methylprednisolone, Meticorten (t), Mitomycin, Mitomycin-C (o), Mitoxantrone, M-Prednisol (t), MTC (o), MTX (o), Mustargen (t), Mustine, Mutamycin (t), Myleran (t),
Mylocel (t), Mylotarg (t), Navelbine (t), Nelarabine, Neosar (t), Neulasta (t), Neumega (t), Neupogen (t), Nexavar ®, Nilandron (t), Nilutamide, Nipent ®, Nitrogen Mustard (o), Novaldex (t), Novantrone (t), Octreotide, Octreotide acetate (o), Oncospar (t), Oncovin (t), Ontak (t), Onxal (t), Oprevelkin, Orapred (t), Orasone (t), Oxaliplatin, Paclitaxel, Paclitaxel Protein-bound, Pamidronate, Panretin (t), Paraplatin (t), Pediapred (t), PEG Interferon, Pegaspargase, Pegfilgrastim, PEG-INTRON (t), PEG-L-asparaginase, PEMETREXED, Pentostatin, Phenylalanine Mustard (o), Platinol (t), Platinol-AQ (t), Prednisolone, Prednisone, Prelone (t), Procarbazine, PROCRIT ®, Proleukin (t), Prolifeprospan 20 with Carmustine implant (t), Purinethol (t), Raloxifene, Revlimid ®, Rheumatrex (t), Rituxan (t), Rituximab, Roferon-A®, (interferon alfa-2a) Rubex (t), Rubidomycin hydrochloride (t), Sandostatin ®, Sandostatin LAR (t), Sargramostim, Solu-Cortef (t), Solu-Medrol (t), Sorafenib, STI-571, Streptozocin, SU11248, Sunitinib, Sutent ®, Tamoxifen, Tarceva ®, Targretin (t), Taxol ®, Taxotere (t), Temodar ®, Temozolomide, Teniposide, TESPA (o), Thalidomide, Thalomid ®, TheraCys (t), Thioguanine, Thioguanine Tabloid (t), Thiophosphoamide (o), Thioplex (t), Thiotepa, TICE ®, Toposar (t), Topotecan, Toremifene, Tositumomab, Trastuzumab, Tretinoin, Trexall (t), Trisenox (t), TSPA (o), VCR (o), Velban (t), Velcade ®, VePesid (t), Vesanoid (t), Viadur (t), Vidaza (t), Vinblastine, Vinblastine Sulfate (o), Vincasar Pfs (t), Vincristine, Vinorelbine, Vinorelbine tartrate (o), VLB (o), VM-26 (o), VP-16 (t), Vumon (t), Xeloda ®, Xyotax, Zanosar (t), Zevalin TM, Zinecard (t), Zoladex ®, Zoledronic acid, and Zometa ®.

Another drugs used in cancer-therapy (mostly as chemotherapeutics) are:
(as trademarks): Aldara, Alimta, Androcur, Arimidex, Borea, Caelyx, Campto, Casodex, Decapeptyl, Eloxatin, Eutirox, Faslodex, Femara, Gemzar, Gonapeptyl, Grisetin, Herceptin, Isovorin, Lysodren, Megefren, Metvix, Navelbine, Novaldex, Novantrone, Paraplatin, Procrin, Prostacur, Suprefact, Tamoxifeno Funk, Taxol, Taxotere, Testex, Elmu/Prolongatum, Tomudex, Utefos, Vepesid, Xeloda, Zoladex;
(as active compounds): Anastrozole, Bicalutamide, Busereline, Capecetabine, Cisplatin, Carboplatin, Desoxorubicin, Docetaxel, Etoposid, Fulvestrant, Gemcitabine, Gosereline, Irinotecan, Letrozole, Leuproreline, Megestrol, Mitotane, Mitoxantrone, Oxaliplatin, Paclitaxel, Pemetrexed, Raltitrexed, Tamoxiphen, Tegafur, Triptoreline, Vincristine, Vinblastine, Vinorelbine, and Vindesine.

In a preferred embodiment of the invention, the chemotherapeutic drug is selected from taxanes, vinca alkaloids and drugs derived form platinum. Preferably, the chemotherapeutic drug is selected from paclitaxel, oxaliplatin and vincristine.

In a more preferred embodiment of the invention, the chemotherapeutic drug is Paclitaxel. Paclitaxel (Taxol®) is one of the most effective and commonly used antineoplastic drugs for the treatment of solid tumours. It has two serious side effects, myelosupression and peripheral neurotoxicity. The granulocyte colony-stimulating factor effectively counteracts the neutropenia in most patients. However, there are no acceptable therapies to prevent or minimize the nerve damage, making neurotoxicity a significant dose-limiting side effect (Rowinsky et al., Semin. Oncol., 1993a 29 4 Suppl. 3, 1-15; Rowinsky et al., J. Clin. Oncol., 1993b, 11(10), 2010-20; Wasserheit et al., J. Clin. Oncol., 1996, 14(7), 1993-9; Gordon et al., J. Clin. Oncol., 1997, 15(5), 1965-73; Mielke et al., Eur. J. Cancer, 2006, 42(1), 24-30). Paclitaxel-induced neurotoxicity typically presents as a sensory neuropathy, with the most common complaints being numbness, tingling, burning pain and cold allodynia (Rowinsky et al., Semin. Oncol., 1993a 29 4 Suppl. 3, 1-15; Chaudhry et al., Ann. Neurol., 1994, 35(3), 304-11; Forsyth et al., J. Neurooncol., 1997, 35(1), 47-53; Dougherty et al., Pain, 2004, 109(1-2), 132-42). Sensory symptoms usually start symmetrically in the feet, but sometimes appear simultaneously in both hands and feet (Rowinsky et al., Semin. Oncol., 1993a 29 4 Suppl. 3, 1-15; Quasthoff and Hartung, J. Neurol., 2002, 249(1), 9-17; Mielke et al., Eur. J. Cancer, 2006, 42(1), 24-30). A clinically significant number of patients with paclitaxel-induced neuropathy experience neuropathic pain. For example, in a study of 27 patients treated with paclitaxel doses of 135, 175 and 250-300 mg/m², neuropathic symptoms occurred in 50, 79 and 100% of patients, progressing to dose-limiting neurotoxicity in 0, 21 and 71% of patients, respectively (Postma et al., Ann. Oncol., 1995, 6(5), 489-94).

In another more preferred embodiment of the invention, the chemotherapeutic drug is Oxaliplatin.

Preferred combinations of the invention comprises the combination of (S)- 4,5,6,7-tetrahydro-4-(2-(morpholin-4-yl)ethyl)-1-phenyl-1H-indazole hydrochloride with a chemotherapeutic drug selected from Paclitaxel, Oxaliplatin and Vincristine.

A more preferred combination of the invention comprises the combination of (S)-4,5,6,7-tetrahydro-4-(2-(morpholin-4-yl)ethyl)-1-phenyl-1H-indazole hydrochloride with Paclitaxel.

Another preferred combinations of the invention comprises the combination of (R)-4,5,6,7-tetrahydro-4-(2-(morpholin-4-yl)ethyl)-1-phenyl-1H-indazole hydrochloride with a chemotherapeutic drug selected from Paclitaxel, Oxaliplatin and Vincristine.

In this case, a more preferred combination of the invention comprises the combination of (R)- 4,5,6,7-tetrahydro-4-(2-(morpholin-4-yl)ethyl)-1-phenyl-1H-indazole hydrochloride with Paclitaxel.

The combination of the invention may be formulated for its simultaneous, separate or sequential administration, with at least a pharmaceutically acceptable carrier, additive, adjuvant or vehicle. This has the implication that the combination of the two active compounds may be administered:
- as a combination that is being part of the same medicament formulation, the two active compounds being then administered always simultaneously.
- as a combination of two units, each with one of the active substances giving rise to the possibility of simultaneous, sequential or separate administration.

In a particular embodiment, the sigma ligand of formula (I) is independently administered from the chemotherapeutic drug (i.e in two units) but at the same time.

In another particular embodiment, the sigma ligand of formula (I) is administered first, and then the chemotherapeutic drug is separately or sequentially administered.

These particular ways of administration are preferably used to prevent the pain developing as a consequence of chemotherapy.

In yet another particular embodiment, the chemotherapeutic drug is administered first, and then the sigma ligand of formula (I) is administered, separately or sequentially, as defined.

This particular way of administration is used to treat the pain developing as a consequence of chemotherapy.

The auxiliary materials or additives for the formulation of the compounds of formula (I) can be selected among carriers, excipients, support materials, lubricants, fillers, solvents, diluents, colorants, flavour conditioners such as sugars, antioxidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical combination in accordance with the invention can be adapted to any form of administration, be it orally or parenterally, for example pulmonar, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application.

Suitable preparations for oral applications are tablets, pills, chewing gums, capsules, granules, drops or syrups.

Suitable preparations for parenteral applications are solutions, suspensions, reconstitutable dry preparations or sprays.

The combination of the invention may be formulated as deposits in dissolved form or in patches, for percutaneous application.

Skin applications include ointments, gels, creams, lotions, suspensions or emulsions.

Suitable form of rectal application is by means of suppositories.

As mentioned above, the compound of formula (I) is suited for its use in the prevention or treatment of pain induced by chemotherapy. Preferably, the pain is induced by a chemotherapeutic drug selected from taxanes, vinca alkaloids and drugs derived from platinum. Preferably, the chemotherapeutic drug is selected from paclitaxel, oxaliplatin and vincristine, more preferably it is selected from paclitaxel or oxaliplatin, even more preferably it is paclitaxel.

In addition, the combination of at least one compound of general formula (I) and at least one chemotherapeutic drug is suited for its use in the prevention or treatment of pain developing as a consequence of chemotherapy. This combination for its use in the prevention treatment of pain can be administered simultaneously, separately or sequentially.

Therefore, another aspect of the invention refers to a combination as defined above for its use in the prevention or treatment of pain induced by chemotherapy.

In a particular embodiment, said combination is used in the prevention of pain induced by chemotherapy.

In another particular embodiment, said combination is used in the treatment of pain induced by chemotherapy.

In a particular embodiment of the present invention, the pain is peripheral neuropathic pain, allodynia, causalgia, hyperalgesia, hyperesthesia, hyperpathia, neuralgia, neuritis or neuropathy. More preferably, the pain is cold-allodynia or mechanical allodynia.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention this term is to be treated as synonymous to "Neurogenic Pain" which is defined by the IASP as "pain initiated or caused by a primary lesion, dysfunction or transitory perturbation in the peripheral or central nervous system". Neuropathic pain according to this invention is restricted to the neuropathic pain resulting from chemotherapy, meaning caused by the use of a chemotherapeutic drug in chemotherapy. The most likely cause of this pain is the chemotherapeutic drug neurotoxicity, and more specifically, its peripheral neurotoxicity.

According to the IASP "allodynia" is defined as "a pain due to a stimulus which does not normally provoke pain" (IASP, Classification of chronic pain, 2nd Edition, IASP Press (2002), 210). According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful" (IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

According to the IASP "neuralgia" is defined as "pain in the distribution of a nerve or nerves" (IASP, Classification of chronic pain, 2nd Edition, IASP Press (2002), 212).

According to the IASP "neuritis" is defined as "inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

In one embodiment of the invention it is preferred that the compound of formula (I) is used in therapeutically effective amounts. The physician will determine the dosage of the present therapeutic agents which will be most suitable and it will vary with the form of administration and the particular compound chosen, and furthermore, it will vary with the patient under treatment, the age of the patient, the type of cancer and pain being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

For example, the dosage regime that must be administered to the patient will depend on the patient's weight, the type of application, the condition and severity of the disease. A preferred dosage regime of comprises an administration of a compound of formula (I) within a range of 0.5 to 100 mg/kg and of the chemotherapeutic drug from 0.15 to 15 mg/kg and it is administered daily in one or several doses.

The following examples and figures are merely illustrative of certain embodiments of the invention and cannot be considered as restricting it in any way.

### Examples

### Example 1. Assessment of the preventive and curative antiallodynic effect of compounds 1a and 1 b in a model of paclitaxel-induced neuropathy in rats.

### Common procedures

Experiments were performed in CD-1 mice (Charles River, U.S.A.) with at least n = 10/experimental group. Mice weighing 25-30 g were used. The animals were housed in colony cages with free access to food and water prior to the experiments. They were maintained in temperature- and light-controlled rooms (22 ± 1 °C, lights on at 08.00 h and off at 20.00 h, air replacement every 20 min). Testing took place during the light phase (from 9.00 h to 15.00 h).

Paclitaxel was dissolved in a solution made up of 50% Cremophor EL and 50% absolute ethanol to obtain a concentration of 6 mg/ml. This paclitaxel solution was conserved at -20 °C during a maximum of 14 days and was diluted in normal saline (NaCl 0.9%), just before administration, to a final concentration of 2 mg/10 ml. The vehicle of paclitaxel was diluted at the time of injection with saline (NaCl 0.9%) in the same proportion as the paclitaxel solution.

Paclitaxel (2 mg/kg) was administered intraperitoneously (i.p.), in a volume of 10 ml/kg, once per day for five consecutive days. Therefore, the cumulative dose was 10 mg/kg per mouse. In the control group the vehicle of paclitaxel was administered following the same schedule.

Compounds **1a** and **1b** were dissolved in normal saline just before administration and applied at doses of 32 or 64 mg/kg subcutaneously.

The effects of compounds 1a and 1b on paclitaxel-induced neuropathic pain were examined in two different ways. To evaluate the effect of the sigma receptor antagonists compounds 1a and 1b on the development of paclitaxel-induced pain, the animals received an s.c. injection of compound 1a or compound 1b 30 min before each i.p. injection of paclitaxel for five consecutive days. The response of the animals to the different nociceptive stimuli was tested subsequently during 2-4 weeks, depending on the test (see below), without any additional treatment. Each animal was tested only in one nociceptive model. To test the effect of compounds 1a and 1b on the *expression* of paclitaxel-induced pain, a single compound 1 a or 1 b injection was performed on day 10, a day of maximum expression of mechanical allodynia or cold allodynia (see figures for details). Each animal received only one dose of compound 1 a or 1b and was tested in only one nociceptive model.

**Procedure for assessment of cold allodynia.** Cold allodynia was tested as previously described by Smith et al. in Life Sci., 2004, 74(21), 2593-604, by gently touching the plantar skin of the hind paws with an acetone bubble formed with a syringe connected to a thin polyethylene tube. The mice were housed and habituated for 30 min in transparent plastic boxes (7 x 7 x 13 cm) with a floor made of wire mesh. After the adaptation period, acetone was applied alternately three times to each paw at intervals of 30 s, and the duration and frequency of licking or biting were recorded. A small mirror was placed behind the chambers to allow clear observation of the paws. The time spent licking or biting the paw was recorded by a stopwatch and represented as the cumulative time of licking/biting in the six measurements. Since licking persisting more than 10 s in the experiments was very unusual, a cut-off time of 10 s was used for each trial.

To test the effect of compounds 1a and 1b on the development of cold allodynia, the animals were tested previously to the paclitaxel administration (pretreatment value, 3 days before first paclitaxel-treatment) and on different days (days 7, 10, 14, 17, 21 and 24) after the first paclitaxel or vehicle injection. Compounds 1 a and 1 b or their vehicles were injected s.c. 30 min before each of the 5 paclitaxel i.p. injections. Once more the animals were tested previously to the paclitaxel/compound 1a or paclitaxel/compound 1b administration (pretreatment value, 3 days before first paclitaxel/compound 1a or paclitaxel/compound 1b treatment) and on different days (days 7, 10, 14, 17, 21 and 24) after the first paclitaxel/compound 1a or paclitaxel/compound 1b or vehicle injection. The effect of compound 1 a and compound 1 b on the expression of paclitaxel-induced cold allodynia was evaluated on day 10, because the maximum allodynic effect was observed on that day. Therefore, the day 10, after the habituation period to the apparatus, baseline latencies were recorded, 30 min later compound 1a, compound 1 b or saline was injected s.c. and paw withdrawal latencies were assessed again 30, 60, 90, 120 and 180 minutes after the injection. Around 33 % of the control animals treated with paclitaxel did not show cold allodynia; therefore, it was differentiated between 'responders' and 'non-responders' mice in this test. The 'non-responders' mice were easily identified because they spent less than 2 s licking/biting the paw stimulated with acetone on days 7 and 10 after paclitaxel administration. The 'non-responder' animals were not used to test the effect of compounds compound 1 a and 1 b on the expression of cold allodynia since they do not express enough cold allodynia.

### Results

### A) Effects of compounds 1a and 1b on the development of paclitaxel-induced cold- allodynia.

Mice were treated once daily from days 1 to 5 with an s.c. injection of compound 1 a (64 mg/kg), compound 1 b (64 mg/kg) or saline 30 min before each i.p. injection of paclitaxel (2 mg/kg). The response was recorded in each animal 3 days before (PRE) and on different days after the first injection of paclitaxel + compound 1a, paclitaxel + compound 1 b or paclitaxel + saline. The pre-treatment values in the acetone test were similar in the different experimental groups (paclitaxel + saline, paclitaxel + compound 1a and paclitaxel + compound 1b) (Figs. 1 and 2). This indicates that there were no differences in the basal response to acetone in the different experimental groups.

The group of animals in which paclitaxel (i.p.) and saline (s.c.) were co-administered during days 1 to 5 showed a significant increase on duration of paw licking/biting (Fig. 1 and 2) in the acetone test, which began on day 7 and became maximum on day 14 after the first injection. On the other hand, in the animals which receive an s.c. dose of compound 1a or compound 1b (both at a dose of 64 mg/kg) 30 min before each i.p. paclitaxel dose during days 1 to 5, the duration of acetone-induced paw licking/biting was reduced. There were statistically significantly differences between the values obtained in paclitaxel + saline and paclitaxel + compound 1a (64 mg/kg) groups, from day 7 to day 21, when duration of acetone-induced licking/biting was analyzed (Fig.1). Similarly, there were significantly lower values of acetone-induced licking/biting in the paclitaxel + compound 1b (64 mg/kg) group in comparison to the paclitaxel + saline group at days 7, 14 and 21 after the first dose of paclitaxel (Fig. 2).

Therefore, co-administration of paclitaxel (2 mg/kg, i.p.) and the sigma-1 receptor antagonists compound 1a or 1b (both 64 mg/kg, s.c.) inhibited the development of cold allodynia induced by paclitaxel.

### B) Effects of co-administration of paclitaxel-vehicle and compound 1a or compound 1 b on the response to acetone

Mice were treated once daily from days 1 to 5 with an s.c. injection of compound 1a, compound 1b (64 mg/kg) or saline 30 min before each i.p. injection of paclitaxel vehicle. The response was recorded in each animal 3 days before (PRE) and on different days after the first injection of paclitaxel vehicle + compound 1a, paclitaxel vehicle + compound 1b or paclitaxel vehicle + saline. As expected, the group of animals treated with paclitaxel-vehicle did not develop cold allodynia (i.e. they did not show an increase in the duration of acetone-induced paw licking/biting) when evaluated during 24 days after the first dose of paclitaxel-vehicle was administered (Figs. 3 and 4).

Co-administration during days 1 to 5 of paclitaxel-vehicle i.p. and the sigma-1 receptor antagonists on study, compound 1a (64 mg/kg, s.c.) and compound 1b (64 mg/kg, s.c.), did not significantly modify the response of the animals to acetone in comparison to that found in animals co-administered with paclitaxel-vehicle and saline (Figs. 3 and 4).

### C) Effects of compounds 1a and 1 b on the expression of paclitaxel-induced cold allodynia

Animals were treated once daily from days 1 to 5 with paclitaxel i.p., and on day 10 received a single s.c. injection of compound 1 a, compound 1 b or saline. The duration of hind paw licking/biting was recorded in each animal 3 days before (PRE) and 10 days after the first injection of paclitaxel. On that day, duration of hind paw licking/biting was recorded immediately before (time 0) and at several times (60, 120 and 180 min) after the injection of compound 1 a, compound 1 b or saline. As expected, all the groups of animals treated with paclitaxel developed cold allodynia (manifested by an increase in the duration of acetone-induced paw licking/biting) when evaluated 10 days after the first dose of paclitaxel was administered (Figs. 5 and 6).

A single s.c. injection of saline on day 10 did not significantly modify the expression of paclitaxel-induced cold allodynia during the following 3 h (Figs. 5 and 6). On the other hand, an acute treatment with compound 1a (32-64 mg/kg, s.c.) dose-dependently inhibited the expression of cold allodynia induced by paclitaxel (Fig. 5). The effect of compound 1a was statistically significant (in comparison with the saline-treated group) at all times evaluated from 60 to 180 min after the administration of the drug. Similarly, an acute injection of compound 2 (32-64 mg/kg, s.c.) also dose-dependently reduced the duration of acetone-induced licking/biting in animals when evaluated 10 days after the first dose of paclitaxel was administered (Fig. 6).

Therefore, the sigma-1 receptor antagonists compound 1a or 1b (both at 32-64 mg/kg, s.c.) inhibited the expression of cold allodynia induced by paclitaxel

### D) Effects of compounds 1a and 1b on the response to acetone of paclitaxel-vehicle treated mice

Animals were treated once daily from days 1 to 5 with paclitaxel-vehicle i.p., and on day 10 received a single s.c. injection of compound 1 a, compound 1b or saline. The duration of hind paw licking/biting was recorded in each animal 3 days before (PRE) and 10 days after the first injection of paclitaxel-vehicle. On that day, duration of hind paw licking/biting was recorded immediately before (time 0) and at several times (60, 120 and 180 min) after the injection of compound 1a, compound 1b or saline. As expected, the animals treated with paclitaxel-vehicle did not develop cold allodynia (i.e. the duration of acetone-induced paw licking/biting did not increase) when evaluated 10 days after the first dose of paclitaxel-vehicle was administered (Figs. 7 and 8).

An acute treatment with compounds 1 a and 1 b (32 or 64 mg/kg, s.c.) did not affect the response to acetone of control (paclitaxel-vehicle treated) animals (Figs. 7 and 8).

## Claims

1. A combination of at least one sigma ligand and at least one chemotherapeutic drug for simultaneous, separate or sequential administration, wherein the sigma ligand has the general formula (I): 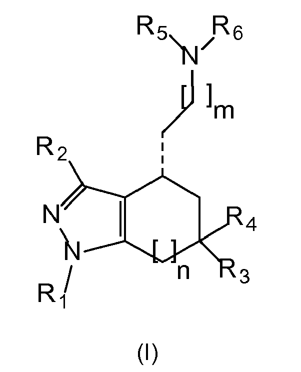 wherein
R₁ is selected from the group formed by hydrogen, susbtituted or unsubstituted alkyl, susbtituted or unsubstituted cycloalkyl, susbtituted or unsubstituted heterocyclyl, susbtituted or unsubstituted aryl, susbtituted or unsubstituted arylalkyl, and susbtituted or unsubstituted, aromatic or non-aromatic heterocyclylalkyl;
R₂ is selected from the group formed by hydrogen, susbtituted or unsubstituted alkyl, susbtituted or unsubstituted cycloalkyl, susbtituted or unsubstituted alkoxy, susbtituted or unsubstituted aryl, susbtituted or unsubstituted, aromatic or non-aromatic heterocyclyl, susbtituted or unsubstituted arylalkyl, and susbtituted or unsubstituted heterocyclylalkyl;
R₃ and R₄ are independently selected from the group formed by hydrogen, susbtituted or unsubstituted alkyl, susbtituted or unsubstituted cycloalkyl, susbtituted or unsubstituted, aromatic or non-aromatic heterocyclyl, susbtituted or unsubstituted aryl, susbtituted or unsubstituted arylalkyl and susbtituted or unsubstituted heterocyclylalkyl or, together, R₃ and R₄ form a 3 to 6 substituted or unsubstituted member ring;
R₅ and R₆ are independently selected from the group formed by hydrogen, susbtituted or unsubstituted alkyl, susbtituted or unsubstituted cycloalkyl, susbtituted or unsubstituted, aromatic or non-aromatic heterocyclyl, susbtituted or unsubstituted aryl, susbtituted or unsubstituted arylalkyl and susbtituted or unsubstituted heterocyclylalkyl or, R₅ and R₆ together, form a substituted or unsubstituted heterocyclyl having 3 to 7 atoms in the ring;
n is selected from 0, 1 and 2;
m is selected from 0, 1, 2, 3 and 4;
the dotted line ----- is either a single or a double bond;
or a pharmaceutically acceptable salt, isomer, tautomer, prodrug or solvate thereof.

2. A combination according to claim 1, wherein R₁ is selected from methyl, t-butyl, cyclohexyl and phenyl.

3. A combination according to claims 1 or 2, wherein R₂ is H or alkyl.

4. A combination according to any of the preceding claims, wherein R₃ and R₄ are both hydrogen or alkyl.

5. A combination according to any of the preceding claims, wherein the dotted line is a single bond.

6. A combination according to any preceding claims, wherein R₅ and R₆ together form a morpholine-4-yl group.

7. A combination according to any preceding claims, wherein the sigma ligand of formula (I) is selected from (S)- 4,5,6,7-tetrahydro-4-(2-(morpholin-4-yl)ethyl)-1-phenyl-1H-indazole hydrochloride and (R)- 4,5,6,7-tetrahydro-4-(2-(morpholin-4-yl)ethyl)-1-phenyl-1H-indazole hydrochloride.

8. A combination according to any of the preceding claims, wherein the chemotherapeutic drug is selected from taxanes, vinca alkaloids and drugs derived from platinum.

9. A combination according to any of the preceding claims, wherein the chemotherapeutic drug is selected from the group formed by paclitaxel, oxaliplatin and vincristine.

10. A combination according to any preceding claims, wherein the combination comprises a sigma ligand of formula (I) selected from (S)- 4,5,6,7-tetrahydro-4-(2-(morpholin-4-yl)ethyl)-1-phenyl-1H-indazole hydrochloride and (R)- 4,5,6,7-tetrahydro-4-(2-(morpholin-4-yl)ethyl)-1-phenyl-1H-indazole hydrochloride; and paclitaxel.

11. A combination as defined in any of claims 1 to 10 for its use in the manufacture of a medicament.

12. A combination as defined in any of claims 1 to 10 for its use in the prevention or treatment of pain induced by chemotherapy.

13. A combination according to claim 12, wherein the pain is selected from peripheral neuropathic pain, allodynia, causalgia, hyperalgesia, hyperesthesia, hyperpathia, neuralgia, neuritis and neuropathy.

14. A compound of formula (I) as defined in any of claims 1 to 7, or a pharmaceutically acceptable salt, estereoisomer, tautomer, prodrug or solvate thereof, for its use in the treatment or prevention of pain induced by chemotherapy.

15. A compound according to claim 14, for its use in the treatment or prevention of pain induced by a chemotherapeutic drug selected from taxane, vinca alkaloid or platinum derivatives.

16. A coumpound according to claim 14, or a pharmaceutically acceptable salt, estereoisomer, tautomer, prodrug or solvate thereof, for its use in the prevention of pain induced by chemotherapy.

17. A compound according to claims 14, 15 or 16, wherein the pain is selected from peripheral neuropathic pain, allodynia, causalgia, hyperalgesia, hyperesthesia, hyperpathia, neuralgia, neuritis and neuropathy.

18. Us of a compound of formula (I) as defined in any of claims 1 to 7, or a pharmaceutically acceptable salt, estereoisomer, tautomer, prodrug or solvate thereof, in the manufacture of a medicament for the treatment or prevention of pain induced by chemotherapy.

19. A method of treatment of a patient suffering from pain induced by chemotherapy, or likely to suffer pain as a result of a chemotherapeutic treatment, which comprises administering to the patient in need of such a treatment or prophylaxis a therapeutically effective amount of a sigma ligand of formula (I) as defined in any of claims 1-7.
